# EUROPÄISCHE PATENTSCHRIFT

(11) **EP 2 104 520 B1**
(45) Veröffentlichungstag und Bekanntmachung des Hinweises auf die Patenterteilung: **31.03.2010**
(21) Anmeldenummer: 07857884.6
(22) Anmeldetag: 19.12.2007
(51) Int. Cl.: A61L 27/34, A61L 27/54, A61L 31/06, A61L 31/14, A61L 31/16, A61L 17/00

(54) **VERFAHREN ZUR HERSTELLUNG EINES PRODUKTES MIT POLYMERMATRIX, DARAUS BESTEHENDE IMPLANTATE SOWIE DEREN VERWENDUNG**
METHOD FOR PRODUCING A PRODUCT HAVING A POLYMER MATRIX, IMPLANTS MADE THEREOF AND USE THEREOF
PROCEDE DE FABRICATION D'UN PRODUIT COMPORTANT UNE MATRICE POLYMERE, DES IMPLANTS ISSUS DE CELLE-CI AINSI QUE SON UTILISATION

(30) Priorität: 20.12.2006 DE 102006060958
(43) Veröffentlichungstag der Anmeldung: 30.09.2009
(73) Patentinhaber: JENNISSEN, Herbert, 50858 Köln (DE)
(72) Erfinder: JENNISSEN, Herbert, 50858 Köln (DE); CHATZINIKOLAIDOU, Maria, 71500 Heraklio-Kreta (GR)
(74) Vertreter: Nobbe, Matthias
(86) Internationale Anmeldenummer: PCT/EP2007/064263
(87) Internationale Veröffentlichungsnummer: WO 2008/074845

(56) Entgegenhaltungen:
- WO-A-2004/105824
- US-A- 4 563 489
- US-A1- 2005 065 214
- KANCZLER ET AL: "Supercritical carbon dioxide generated vascular endothelial growth factor encapsulated poly(dl-lactic acid) scaffolds induce angiogenesis in vitro" BIOCHEMICAL AND BIOPHYSICAL RESEARCH COMMUNICATIONS, ACADEMIC PRESS INC. ORLANDO, FL, US, Bd. 352, Nr. 1, 30. November 2006 (2006-11-30), Seiten 135-141, XP005727934 ISSN: 0006-291X

## Beschreibung

Die Erfindung betrifft ein Verfahren zur Herstellung eines Produktes mit Polymermatrix, danach erhältliche Produkte sowie deren Verwendung.

Die Einbringung biologisch aktiver Proteine wie z.B. Wachstumsfaktoren in künstliche implantierbare Polymere mit nachfolgender Freisetzung im Gewebe unter Erhalt der biologischen Aktivität ist ein noch ungelöstes Problem in der Biomaterial- und Implantatforschung. Ein sehr vielversprechendes Polymer, das sich zur Lösung der Probleme anbietet, ist das biologisch abbaubare Polylaktid. So ist schon früh versucht worden Wachstumsfaktoren u.a. BMP-2 in Polylaktide einzubringen. So konnten beispielsweise Weber et al. [(2002) Slow and continuous application of human recombinant bone morphogenetic protein via biodegradable poly(lactide-co-glycolide) foamspheres. Int. J. Oral Maxillofac. Surg., 31, 60-65] rhBMP-2 in einer Menge von 170 µg/g Poly(Laktid-co-Glycolid) (PLGA) in das PLGA unter drastischen Bedingungen (Dichlormethan, 6M. Harnstoff) einkapseln, jedoch erfolgte die fast vollständige Freisetzung des eingekapselten rhBMP-2 bereits innerhalb von ca. 10 Tagen.

Kanczler et al. [(2007) Supercritical carbon dioxide generated vascular endothelial growth factor encapsulated poly (DL-lactic acid) scaffolds induce angiogenesis in vitro. Biochemical and Biophysical Research Communications 352, 135-141] beschreibt die Verkapselung von aktivem VEGF in poröse Poly-(D,L)-Milchsäure-Gerüste zur Aktivierung der Angiogenese.

Poly(D,L-Lactid) ist ein amorpher Polyester der D- und L-Milchsäure mit einer Glastemperatur von ca. 57 °C. Auf Grund des Fehlens der Kristallinität besitzt PDLLA eine niedrigere Festigkeit und einen niedrigeren Elastizitätsmodul als kristallines Poly(L-Lactid) (PLLA). Besondere Vorzüge des Gewebeersatzmaterials Polylactid sind die Festigkeit über mehrere Wochen, die Abbaubarkeit und die Entstehung des physiologischen Monomers Milchsäure als endgültigem Abbauprodukt.

Der PLLA-Abbau in vitro und in vivo erfolgt in fünf Phasen:

| | |
|---|---|
| Phase 1: | Hydratation; |
| Phase 2: | Depolymerisation ohne Masseverlust (10-20 Wochen, neue Knochenbälkchen); |
| Phase 3: | Masseverlust; |
| Phase 4: | Absorption (2-5 Jahre, Aufnahme von Fragmenten durch Phagozyten); |
| Phase 5: | Elimination (Lactat wird in Pyruvat umgewandelt und verstoffwechselt). |

Im Stand der Technik ist zur Aufschäumung von Polylactiden aus der Sheridan, M. H., Shea, L. D., Peters, M. C., & Mooney, D. J. (2000) J. Control Release, 64, 91-102 bekannt, dass trockene Pulver von Polylactid-co-Glycolid und lyophilisiertem Blutgefäßwachstumsfaktor VEGF gemischt und aufgeschäumt (59 bar) werden, wobei der Schaum fragmentiert. In anderen Versuchen wurde eine offenporige Struktur durch Schäumung mit NaCl hergestellt. Die Freisetzungsraten schwankten zwischen 2 und 70 Tagen.

Seitens der Erfinder wurde nun gefunden, dass stabile Produkte, insbesondere Schäume mit eingebundenem Knochenwachstumsfaktor, aus einer Mischung aus einem Polylactid mit einer Lösung mindestens eines Knochenwachstumsfaktors der BMP-Klasse, insbesondere BMP-2, hergestellt werden können.

Die Erfindung betrifft daher ein Verfahren zur Herstellung eines Produktes mit Polymermatrix, das die folgenden Schritte umfasst:
a) Mischen eines pulverförmigen biologisch abbaubaren Polymers, ausgewählt aus Polymilchsäure, Polyglykolsäure oder deren Copolymeren, mit einer wässrigen Lösung eines Knochenwachstumsfaktors der BMP-Klasse mit einer Konzentration in der wässrigen Lösung, die 0,5 mg bis 10 mg Knochenwachstumsfaktor pro Gramm an biologisch abbaubarem Polymer im Produkt entspricht, und mit einem pH-Wert von 4 bis 5, oder mit einem PH-Wert von 9,5 bis 10,5 zu einer Schlämme in einer Menge, dass die wässrige Lösung nahezu vollständig von dem pulverförmigen Polymer aufgesaugt wird;
b) Trocknen der in Schritt a) erhaltenen Schlämme;
c) Einbringen des Produktes aus Schritt b) in eine Formgebungsvorrichtung; und
d) Formen des in die Formgebungsvorrichtung eingebrachten Produktes in eine Applikationsform zur Förderung des Zellwachstums..

Die Erfinder haben dabei als eines der wesentlichen Elemente der Erfindung festgestellt , dass eine Schlämme gebildet wird, d.h. es ist kein Flüssigkeitsüberstand neben dem Pulver vorhanden. Das primäre "Trocknen" der Schlämme wird dabei mehr als ein spontanes Trockenwerden auf Grund der Hygroskopie des Polymer-Pulvers wie PDLLA-Pulvers verstanden. Die Hygroskopie bewirkt, daß die wässrige Lösung mit dem z.B. BMP in die Polymer-Partikel aufgesaugt wird und sich vermutlich mit den Polymer-Partikeln feinst und adsorptiv verbindet. In der Regel wird dabei auf ein Gramm Polymer 1,0 bis 3,5 ml der wässrigen Lösung eingesetzt, und das pulverförmige Polymer saugt die wässrige Lösung nahezu vollständig auf, so dass ein etwas feuchtes Pulver verbleibt. Sekundär wird ein Trocknungsprozeß z.B. Lufttrocknung oder Einfrieren mit nachfolgender Lyophilisierung eingesetzt.

Um den Wachstumsfaktors, Cytostatikum, Antibiotikum oder Mischungen davon bei dem Trocknen in der biologischen Aktivität zu erhalten und zu schützen, kann in der wässrigen Lösung ein mehrwertiger Zucker wie Sucrose vorhanden sein.

Das Fehlen des Überstandes oder einer flüssigen Phase in der Schlämme bewirkt, daß nur sehr geringe Mengen nicht im PDLLA Pulver aufgenommenen BMPs zwischen den PDLLA-Partikeln beim Trocknungsprozeß verbleiben. Je mehr flüssige Phase, d.h. partikelfreie Phase, vorhanden ist, desto mehr "Eisbrocken" fallen beim Einfrieren vor der Lyophilisierung an. Wenn diese Eisbrocken trockenen, so fällt extrapartikuläres BMP-2 Pulver an. Nach Vermutung der Erfinder ist es dieses extrapartikuläre BMP, welches später nicht so aufgeschäumt wird, außen auf den Tabletten verbleibt und die "Burst-Phase" bedingt, die seitens der Erfinder nicht angestrebt wird.

Mittels des erfindungsgemäßen Verfahrens ist es nun möglich, ein resorbierbares osteoinduktives Polymermaterial zur verbesserten bzw. beschleunigten Knochenheilung und - neubildung durch Freisetzung des Wachstumsfaktors bei zeitgleicher Degradation eines individuellen präoperativ gefertigten Implantats herzustellen. Dies kann insbesondere durch eine homogene Verteilung des Wachstumsfaktors, insbesondere BMP-2, bei einer hohen Beladungsmöglichkeit mit langdauernder Freisetzung unter Erhalt der biologischen Aktivität erreicht werden.

In dem erfindungsgemäßen Verfahren setzt man vorzugsweise ein pulverförmiges biologisch abbaubares Polymer mit einer Teilchengröße bis 500 µm ein. Dieses biologisch abbaubar Polymer ist Polymilchsäure, Polyglykolsäure oder deren Copolymere.

Um das physiologische Milieu bei dem Abbau des Polymers im Körper zu stabilisieren, kann das pulverförmige biologisch abbaubare Polymer zusätzlich ein pH-Stabilisierungsmittel wie CaCO3, NaHC03 etc. enthalten. Ein Nachteil des Polysäure wie Polylactids ist jedoch die pH-Absenkung um mehrere pH-Einheiten während der Hydrolyse, weshalb dem Polymer auch vorzugsweise Puffersubstanzen (z.B. CaCO₃ oder 80% NagHPO₄/20% NaH₂PO₄) zugesetzt werden können.

In einer weiteren Ausführungsform werden auf ein Gramm des Polymers 1,5 bis 2,5 Milliliter der wässrigen Lösung des Knochenwachstumsfaktors eingesetzt.

Diese wässrige Lösung des Knochenwachstumsfaktors der BMP-Klasse, insbesondere BMP-2, sollte eine Konzentration an Wachstumsfaktor besitzen, die von 0,5 mg bis 10mg Knochenwachstumsfaktor pro Gramm an biologisch abbaubaren Polymeren im Produkt ermöglicht. Eine besonders hohe Beladung des Polymers mit Knochenwachstumsfaktor kann mit einer wässrigen Lösung des Knochenwachstumsfaktors der BMP-Klasse bei einem pH-Wert von 4 bis 5, oder mit einem pH-Wert von 9,5 bis 10,5 erzielt werden.

Wenn die Mischung aus dem Polymer und der wässrigen Lösung des Knochenwachstumsfaktors nicht ausreichend rieselfähig ist, kann zur besseren Weiterverarbeitung das erhaltene Produkt, die Schlämme, lyophilisiert werden.

In einem nächsten Verfahrenschritt kann das Lyophilisat in einen Extruder als Formgebungsvorrichtung eingebracht und mittels des Extruders bei einer Temperatur oberhalb der Glasübergangstemperatur des biologisch abbaubaren Polymers und unterhalb der Denaturierungstemperatur des Knochenwachstumsfaktors zu einem Granulat extrudiert werden. Auf diese Weise wird ein Granulat erhalten, das eine bereits gut homogene Verteilung des Wachstumsfaktor im Polymer besitzt.

Eine noch homogenere Verteilung kann erreicht werden, wenn das das Lyophilisat in einen Autoklaven als Formgebungsvorrichtung eingebracht wird, in dem Autoklaven oberhalb der Glasübergangstemperatur des biologisch abbaubaren Polymers und unterhalb der Zersetzungstemperatur des Knochenwachstumsfaktors einer Druckbegasung mit überkritischem Kohlendioxid unterworfen wird, anschließend der Autoklav druckentlastet wird und das erhaltene schaumförmige Produkt aus dem Autoklaven entnommen wird.

Das so erhaltene schaumförmige Produkt, das auch weiter in die gewünschte Applikationsform geformt werden kann, zeichnet sich durch eine lang andauernde Freisetzung unter Erhalt der biologischen Aktivität aus, die im Stand der Technik nicht bekannt ist.

Die die gewünschte Applikationsform umfasst wie oben ein chirurgisches Befestigungsmittel wie Faden, Stift, Nagel, Schraube oder Niete, eine Platte oder Membran, oder die Verwendung als Beschichtungsmittel für metallische oder keramische Implantate, auf die die das schaumförmige Produkt aufgeschmolzen werden kann.

Die Erfindung betrifft daher auch ein Produkt mit einer Polymermatrix, erhalten nach dem erfindungsgemäßen Verfahren sowie daraus hergestellte Applikationsformen wie ein chirurgisches Befestigungsmittel wie Faden, Stift, Nagel, Schraube oder Niete, eine Platte oder Membran, sowie die Verwendung zur Beschichtung auf Implantaten und die so beschichteten Implantate.

### Herstellungsbeispiel

Erfindungsgemäß wird als ein Ausführungsbeispiel das Polylactid-Calcium Carbonat-Komposit (pH-Stabilisierung) aus 2 g Poly(D,L-Lactid) (200-500 µm PDLLA, Resomer R 207 oder R 208, Boehringer Ingelheim) in 100 ml Chloroform gelöst, mit CaCO₃ (Merck, p.a.) im Verhältnis (w:w = 80:20) nach Vorschrift dispergiert, in 300 ml Ethanol ausgefällt, getrocknet und zu einem Pulver mit einer Korngröße von 200-400 µm mechanisch zermahlen PDLLA-CaCO-1-. Granulat).

Dann kann die Herstellung des PDLLA-CaCO₃-BMP-2 Komposits wie folgt erfolgen: 1 g PDLLA-CaCO₃ Granulat wird in 1.6 ml BMP-2 Lösung (125 µg/ml in ungepufferter 15 mM Sucrose, wobei die BMP-2 Konzentration bis 2 mg/ml in ungepufferter 15 mM Sucrose oder in bei pH 4.5 bzw. pH 10.0 gepufferter 15 mM Sucrose betragen kann, aufgenommen und in einem Potter-Homogenisator mit Teflonpistil manuell mit Drehbewegungen homogenisiert. Während des Homogenisierungsvorganges wurde das Material wieder trocken, so dass am Ende der Homogenisierung wiederum ein Pulver vorlag. Dieses Pulver wurde entweder direkt oder nach Lyophilisierung zu aufgeschäumten Tabletten in einem Begasungsverfahren weiterverarbeitet. Dabei wird das PDLLA-CaCO₃-BMP-2 Pulvergemisch in einer Teflonhohlform (10 x 5 x 2 mm Öffnungen) einer Druckbegasung mit überkritischem CO₂ (100 bar) in einem Autoklaven bei einer Temperatur von ca. 35-55 °C unterworfen. Die Glasübergangstemperatur des PDLLA liegt bei einem Druck von 100 bar bei < -50 °C , d.h. bei 100 °C oberhalb der Glasübergangstemperatur ist das Polylactid thermoplastisch/flüssig und löst das BMP-2. Das PDLLA-CaCO₃-BMP-2 Pulvergemisch wird ca. 2 Stunden bei diesen Bedingungen gehalten (Haltezeit). Innerhalb von 20 Minuten werden dann die Temperatur auf Zimmertemperatur und der Druck auf Umgebungsdruck gesenkt (Dekompression). Unter diesen Bedingungen wird eine bestimmte "Porosität", die durch schnellere Dekompression erhöht werden kann, erhalten. Das BMP-2 muss also unter diesen Bedingungen einen Druck von 100 bar und eine Temperatur von 55 °C aushalten, um biologisch aktiv zu bleiben.

Seitens der Erfinder wurde erstmals ¹²⁵I-markiertes rhBMP-2 eingesetzt, um die einzelnen Schritte der Herstellung und die Eigenschaften aufgeschäumter BMP-2-beladener Polylaktid-Tabletten exakt verfolgen zu können. Es konnte gezeigt werden, dass beispielsweise der Knochenwachstumsfaktor rhBMP-2 in Mengen bis zu 3.4 mg/g Polylaktid inkorporiert werden kann und daß dieses rhBMP-2 mit einer Geschwindigkeitskonstante von 1.6 x 10⁻³ [d] d.h. mit einer Halbwertszeit von - 400 Tagen freigesetzt wird. Dabei ist das freigesetzte rhBMP-2 sowohl *in vitro* als auch *in vivo* aktiv.

### Aktivitätsmessungen

Rekombinantes humanes "bone morphogenetic protein" (rhBMP-2) wurde in E. coli nach bisher beschriebenen Methoden mit einem Reinheitsgrad von > 95% von den Erfindern hergestellt. Die biologische Aktivität des so erhaltenen rhBMP-2 kann mit Hilfe einer Zellkultur (MC3T3-E1 Zellern) bestimmt werden.

Die radioaktive Markierung von rhBMP-2 erfolgte nach der Chloramin-T Methode (¹²⁵I-rhBMP-2), wie für das Protein Ubiquitin im Stand der Technik beschrieben wurde. Die biologische Aktivität des ¹²⁵I-rhBMP-2 bleibt bei dieser Methode voll erhalten.

Die Beladung des biologisch abbaubaren Polymers konnte wie oben erwähnt anhand von PDLLA gezeigt werden. PDLLA (Poly(D,L-Laktid) Resomer 207 und Resomer 208 (mit einem geringfügig unterschiedlichen Polymerisationsgrad) wurde käuflich erworben und in Chloroform aufgelöst. Ein pH-Stabilisierungsmittel wie CaCO₃ kann zur pH-Stabilisierung während der Hydrolyse im Organismus zugesetzt werden. Das PDLLA/CaCO₃-Gemisch kann mit einem organischen Lösungsmittel wie Ethanol gefällt, dann getrocknet, zu einem feinen Pulver gemahlen und gesiebt (Korngröße - 200 µm) werden sowie mit einem Wachstumsfaktor wie rhBMP-2 in einer wässrigen Lösung gemischt werden. Das so erhaltene Kompositmaterial kann dann mit überkritischem CO₂ nach der Methode von Tschakaloff et al. in rechteckigen Tabletten (10 x 5 x 2 mm, ∼35 mg/Tablette) in einem Autoklaven aufgeschäumt werden.

Für Tierversuche wurden runde Tabletten mit Durchmesser von 5 mm und einer Höhe von 2 mm verwendet (∼20 mg/Tablette). Die jeweilige Beladung und die Beladungsausbeute wurden mit Hilfe des ¹²⁵I-rhBMP-2 (siehe oben) bestimmt. Die Dichte der aufgeschäumten Tabletten betrug etwa 0.5 g/cm³.

### Freisetzung von rhBMP-2

Zur Messung der Freisetzungskinetiken wurden aufgeschäumte ¹²⁵I-rhBMP-2 haltige Tabletten in 1.5 ml PBS Puffer (Phosphate Buffered Saline: 137 mM NaCl, 8.1 mM Na₂HPO₄, 2.7 mM KCl, 1.5 mM KH₂PO₄, pH 7.4) in kleinen Reagensröhrchen aufgenommen und unter multiplen Wechseln mit 1.5 ml PBS zur ständigen Durchmischung auf einem rotierenden Rad bei Zimmertemperatur für 107 Tage inkubiert. Zu bestimmten Zeiten (Pufferwechsel) wurden die Tabletten aus den Reagensröhrchen herausgenommen, zweimal in frischem PBS (1.5 ml) gewaschen, in neue Eppendorfgefäße mit frischem PBS-Puffer überführt und dann im Gammazähler (Wizard TM3, Wallac, Finnland) gemessen. Die berechneten Halbwertszeiten wurden entsprechend dem spontanen Zerfall des ¹²⁵I (1/2= 60 d) korrigiert.

### Biologische Aktivitätsmessungen

Die biologische Aktivität des löslichen rhBMP-2 wurde durch Aufnahme der Dosiswirkungskurve (Induktion der alkalischen Phosphatase) mit MC3T3-E1 Zellen gemessen. Die biologische Aktivität wird als Halbaktivierungskonstante (K^{r}₀.₅) angegeben, wobei die Normwerte für die Konstante bei 3-20 nM sowohl für vom Erfinder hergestelltes als auch für das kommerziell erhältliche rhBMP-2 (InductOs, Wyeth) liegen.

### Tierversuche

rhBMP-2 haltige (0.8 - 3.4 mg/g PDLLA, Resomer 207) Tabletten (5 mm Durchmesser x 2 mm Höhe) wurden in einem kritischen Defektheilungsmodell (Schafsrippe) in 5 mm Durchmesser Bohrungen unter Einhaltung der Tierschutzbestimmungen eingesetzt..

### Präparateherstellung

Die Gewebeproben wurden unmittelbar nach der Entnahme vorsichtig vom Weichteilmantel befreit und zur Primärfixierung in phosphatgepufferte Formaldehydlösung (4,5%) eingebracht. Entsprechend einer modifizierten histologischen Technik zur Herstellung unentkalkter Dünnschliffpräparate nach Prof. Donath wurden die Gewebeproben nach Primärfixierung in einer aufsteigenden Alkoholreihe entwässert. Zum Erhalt der PDLLA-Anteile wurden die Proben anschließend in Technovit 7200 VLC eingebettet. Nach Lichtpolymerisation des Einbettungsmaterials wurden von jeder Probe 2 Dünnschliffe mit einer Dicke von 20 µm hergestellt. Zur Oberflächenfärbung der fertig bearbeiteten Schliffpräparate wurde eine 1%ige Thioninlösung verwendet.

### Histomorphometrie

Die histomorphometrische Auswertung erfolgte mittels eines semiautomatischen, an ein Durchlichtmikroskop (Eclipse 800, Nikon Corp., Tokyo, Japan) gekoppelten Bildanalyseprogramms (Lucia 32g/4.51, Laboratory Imaging Ltd., Prag, Tschechische Republik) bei einer 4-fachen Originalvergrößerung. Hierzu wurden standardisierte Messrahmen zentrisch über den Defektbereich mit den darin enthaltenen PDLLA Tabletten positioniert und die Flächenanteile für neugebildetes Knochengewebe (mineralisiert und unmineralisiert) und Leerraum erhoben sowie aus diesen Werten der Anteil an verbliebenem PDLLA berechnet.

### Aufbau einer Isotopenmethode zur Messung der Beladung

Um die Einbringung von rhBMP-2 in PDLLA oder auch PLGA Tabletten möglichst eindeutig, fehlerfrei und mit höchster Empfindlichkeit quantifizieren zu können, wurde die Isotopenmethode der Radioiodierung von rhBMP-2 zum ¹²⁵I-rhBMP-2 gewählt.

Wie oben beschrieben, wurde das PDLLA-Pulver mit rhBMP-2 gemischt und dann in der Hochdruckbegasungsanlage aufgeschäumt. Die Ergebnisse sind in der nachfolgenden Tabelle dargestellt.

**Tabelle 1 -Beladung von PDLLA-Tabletten mit ¹²⁵I-rhBMP-2¹⁾**

| Einwaage ¹²⁵I-rhBMP-2 im Ansatz [mg/g] | Gefundene Beladung mit ¹²⁵I-rhBMP-2 [mg/g] |
|---|---|
| 0.3 | 0.342 ± 0.032 (10) |
| 0.5 | 0.475 ± 0.047 (10) |
| 1.0 | 1.0 ± 0.145 (10) |

| | |
|---|---|
| *¹⁾ Angegeben sind Mittelwert und Standardabweichung, n in Klammern (siehe auch:* Lange, M., Sänger, T., Chatzinikolaidou, M., Laub, M., Jennissen, H.P. (2008) "rhBMP-2 composite of foamed Poly-(D,L-) lactide as drug delivery system for bone tissue engineering" Abstract Commun. 8th World Congress on Biomaterials, May 28-June 1, 2008, Amsterdam, NL*).* | |

Wie aus der Tabelle 1 hervorgeht, konnten Beladungen von 0.34-1.0 mg ¹²⁵I-rhBMP-2 pro g Tablette in guten Ausbeuten erhalten werden. Die Versuche zeigen, daß hier eine effektive Methode zur Beladung der Tabletten entwickelt wurde.

### Freisetzungsversuche

Die wie in der Tabelle 1 hergestellten Tabletten konnten auf Grund der radioaktiven Markierung des rhBMP-2 sogleich für die Freisetzungsversuche verwendet werden. Wie der Erfinder feststellte, führt die Inkubation von ¹²⁵I-rhBMP-2/PDLLA Tabletten in PBS-Puffer pH 7.4 zu einer langsamen Freisetzung des ¹²⁵I-rhBMP-2 über einen Zeitraum von 107 Tagen. Die nichtlineare Anpassung an eine zweiphasige Exponentialfunktion führte zu einer guten Anpassung, so daß die Geschwindigkeitskonstanten und die Halbwertszeiten leicht ermittelt werden konnten. Für den Zeitverlauf ergab sich eine Freisetzung in zwei Phasen, einer initialen sog. Burst-Phase innerhalb der ersten 1-3 Tage mit einer Halbwertszeit von t½ ∼ 0.3-0.5 d und einer zweiten langsamen, prolongierten, monoexponentiellen Hauptphase für den Rest der Beobachtungszeit mit einer Halbwertszeit von t½ ∼ 400-469 d. Aus den langen Halbwertszeiten der zweiten und damit Hauptphase bietet sich der Schluß an, daß es keine Poren für die Freisetzung des ¹²⁵I-rhBMP-2 im aufgeschäumten PDLLA gibt, sondern, daß die Freisetzung erst mit der Hydrolyse bzw. Dekomposition des PDLLA beginnt. Somit ist auch eine Bindung des ¹²⁵I-rhBMP-2 an der Oberfläche der Tablette in größeren Mengen, außer im Falle der Burst-Phase, unwahrscheinlich. Eine Zusammenfassung der kinetischen und statistischen Daten befindet sich in der nachfolgenden Tabelle. Die Geschwindigkeitskonstanten für die Freisetzung des ¹²⁵I-rh-BMP-2 der drei unterschiedlich beladenen PDLLA-Tabletten liegen zwischen k^{b}₋₁ = 1.5-1.7 x 10⁻³ [d⁻¹] oder (k^{b}₋₁ ∼ 2.0 x 10⁻⁸ [s⁻¹]).

**Tabelle 2**

| rh-BMP-2 Beladung [mg/g] | Halbwertszeit von rh-BMP-2 [d] | | Geschwindigkeits-Konstanten der Freisetzung | | [r²] |
|---|---|---|---|---|---|
| | t_{1/2} 1 | t_{1/2} 2 | k^{a}₋₁ [d⁻¹] | k^{b}₋₁ [d⁻¹] | |
| 0.342 ± 0.032 | 0.48 | 404.2 | 1.45 ± 0.53 | 0.00172 ± 0.000154 | 0.966 |
| 0.475 ± 0.047 | 0.27 | 440.4 | 2.59 ± 0.80 | 0.00157 ± 0.000122 | 0.972 |
| 1.0 ± 0.145 | 0.41 | 469.0 | 1.71 ± 0.54 | 0.00148 ± 0.000123 | 0.971 |

| | | | | | |
|---|---|---|---|---|---|
| ²⁾Angegeben sind Mittelwerte und Standardfehler, n = 20. Die Halbwertszeiten wurden entsprechend dem spontanen Zerfall von ¹²⁵I (t_{1/2}= 60 d) korrigiert. Die calcithaltigen Tabletten hatten ein Gewicht von 35 mg. ¹⁾ Angegeben sind Mittelwert und Standardabweichung, n in Klammern (siehe auch: Lange, M., Sänger, T., Chatzinikolaidou, M., Laub, M., Jennissen, H.P. (2008) "rhBMP-2 composite of foamed Poly-(D,L-) lactide as drug delivery system for bone tissue engineering" Abstract Commun. 8th World Congress on Biomaterials, May 28-June 1, 2008, Amsterdam, NL). | | | | | |

Wie in der nachfolgenden Tabelle 3 gezeigt, wurden die absoluten Mengen an freigesetztem ¹²⁵I-rh-BMP-2 (initiale Beladung 1.0 ± 0.145 mg/g) für verschiedene Zeiträume berechnet. In 107 Tagen wurde insgesamt 230 µg ¹²⁵I-rh-BMP-2/g PDLLA d.h. (23% der Gesamtmenge) freigesetzt. In der Burst-Phase waren es innerhalb eines Tages 93 µg/g. Wie in der nachfolgenden Tabelle 3 gezeigt, ist somit eine dreimonatige konstante und sehr effektive Stimulierung des Knochenwachstums möglich.

**Tabelle 3**

| Freigesetzte absolute Mengen an ¹²⁵I-rhBMP-2 aus 10 mit 1.0 mg/g beladenen Tabletten über einen Zeitraum von 107 Tagen³⁾ | | |
|---|---|---|
| Zeitraum | ¹²⁵I-rh-BMP-2 Freisetzung µg/Tag x g | ¹²⁵I-rh-BMP-2 freigesetzte Gesamtmenge µg/g |
| 1. Tag | 93 | 93 |
| 3.-10. Tag | 5.6 | 132 |
| 10.-19. Tag | 1.2 | 142 |
| 19.-48. Tag | 1.7 | 192 |
| 58-107. Tag | 0.8 | 230 |

| | | |
|---|---|---|
| ³⁾Die freigesetzten Mengen an ¹²⁵I-rhBMP-2 wurden an Hand der Daten in und Tab. 2 berechnet. | | |

Interessanterweise wurde über einen sehr langen Zeitraum von 93 Tagen eine konstante Freisetzungsgeschwindigkeit von 0.8-1.2 µg/Tag x g erhalten. Berechnet man nun für eine durchschnittliche Freisetzungsrate von 1.0 µg/Tag x g die Konzentration des freigesetzten rhBMP-2 aus einer 20 mg Tablette (= 20 ng/Tag) in einem Knochendefekt (Ø = 5mm x 2 mm Tiefe) des Volumens von ∼40 µl, so erhält man den Wert -20 nM, was eine fast maximale Stimulierung der MC3T3-T3 Zellen *in vitro* bedeutet.

Die erfindungsgemäß erhaltenen Tabletten (10 x 5 x 2 mm) besaßen ein Gewicht von 40-60 mg/Tablette und enthielten 20-80 µg BMP-2/Tablette. Die Struktur des aufgeschäumten PDLLA ist schwammmatrixartig, und die biologische Aktivität des Knochenwachstumsfaktors wird nahezu unverändert beibehalten.

### Nachweis der biologischen Aktivität von rhBMP-2/PDLLA-Tabletten im Tierversuch.

Wie oben erwähnt, wurden seitens der Erfinder Tierversuche in der Schafsrippe durchgeführt. In den Rippen wurden Lochdefekte (Ø = 5 mm x 2 mm Tiefe) gesetzt in die paßgenau mit BMP-2 beladene PDLLA-Tabletten gleicher Größe (20 mg) eingefügt wurden. Nach 8 Wochen Liegezeit wurden die Schafe getötet und die Rippen entfernt. Die histomorphometrische Auswertung der mit Thioninlösung angefärbten Dünnschliffpräparate erfolgte mittels eines semiautomatischen, an ein Durchlichtmikroskop (Eclipse 800, Nikon) gekoppelten Bildanalyseprogramms (Lucia 32g/4.51, Laboratory Imaging Ltd.) bei einer 4-fachen Originalvergrößerung. Es wurden die Flächenanteile für neugebildetes Knochengewebe (mineralisiert und unmineralisiert) und der Leerraum bestimmt. Als deutlichen Unterschied erkennt man auf den Leerpräparaten (ohne rhBMP-2) nur spärliche punktuelle Areale mit Knocheneubildung, und diese liegen vorzugsweise in der Peripherie der Präparate am Übergang zum ortsständigen Knochen der Defekte oder unmittelbar subperiostal. Im Gegensatz dazu stellt sich die Knochenneubildung in den rh-BMP2 Präparaten deutlich umfangreicher dar. Die multiplen Areale mit Knochenneubildung sind zudem diffus über den gesamten Querschnitt der PDLLA-Tablette verteilt und nicht nur peripher gruppiert. Einhergehend mit einer signifikant stärker ausgeprägten Knochenneubildung findet sich auch ein erhöhter Abbau des PDLLA-Materials mit deutlich zahlreicheren Resorptionsstellen.

Eine quantitative Auswertung der Tierversuche ist in Tab. 4 zusammengefaßt. Es wurden zwei Arten von Kontrollen durchgeführt: Kontrolle 1 in einer Rippe, wo nur Implantate ohne rhBMP-2 eingesetzt waren und Kontrolle 2, bei denen BMP-2 haltige Implantate in der gleichen Rippe vorhanden waren. Wie Tab. 4 zu entnehmen ist, liegen die Kontrollen 2 immer über den Kontrollen 1, was bedeutet, daß das rhBMP-2 in Nachbarschaft zu den Kontrollen 2 bereits zu einer deutlichen Erhöhung des Knochenwachstums in den Präparaten führt. Die Kontrollen 2 wurden daher von den Erfindern nicht als echte Kontrollen anerkannt. Vergleicht man nun die rhBMP-2 haltigen Tabletten (0.8-3.4 mg/g) mit der Kontrolle 1, so findet man eine signifikante Erhöhung des prozentualen Anteils des neugebildeten Knochens (p < 0.05, im ungepaarten, zweiseitigen t-Test). Bei einem Gehalt von 3.4 mg/g war der Anteil neugebildeten Knochens sogar signifikant größer als die Kontrolle 2. Die Inkorporation von rhBMP-2 in aufgeschäumte PDLLA Tabletten führt daher zu einer starken Stimulierung des Knochenwachstums und zu einem Ersatz des PDLLA durch Knochen im Rippenmodell des Schafes nach 8 Wochen.

**Tabelle 4**

| De novo Knochenbildung (Morphometrie) in einem "Critical Size Defect" in Schafsrippen 8 Wochen nach Implantation³⁾. | | | | |
|---|---|---|---|---|
| **Resomer 207** | Knochen, % | *x̅* ± *s* % | P (gegen Kontrolle 1) | P (gegen Kontrolle 2) |
| **Kontrolle 1** | 0,81 | 1.34 ± 0.86 | - | - |
| | 0,87 | | | |
| | 2,34 | | | |
| **Kontrolle2** | 4,40 | 2.93 ± 1.48 | - | - |
| | 2, 95 | | | |
| | 1,43 | | | |
| **0.8 mg/g rh-BMP2** | 5,22 | 7.79 ± 7.50 | 0.2129 | 0.3321 |
| | 16,25 | | | |
| | 1,92 | | | |
| **1.8 mg/g rh-BMP2** | 5,44 | 5.22 ± 2.12 | 0.0426 | 0.1995 |
| | 3,00 | | | |
| | 7,22 | | | |
| **3.4 mg/g rh-BMP2** | 7,58 | 8.18 ± 2.69 | 0.0137 | 0.0413 |
| | 11,12 | | | |
| | 5,85 | | | |

| | | | | |
|---|---|---|---|---|
| ³⁾Angegeben sind Mittelwerte und Standardabweichungen n = 3. Signifikanz ist gegeben bei P < 0.05. Es handelt sich um Tabletten mit einem Durchmesser von 5 mm und 2 mm Dicke. Das Gewicht betrug 20 mg (siehe auch Lange, M., Spassova, E., Laub, M., Schopper, C.; Sänger, T., Stoev, V., Ewers, R., Jennissen, H.P. (2008) "Critical Size Rib Defects in Sheep as a New Model for Testing rhBMP-2 Osteoinductivity of rhBMP-2/PDLLA" Abstract Commun. 8th World Congress on Biomaterials, May 28-June 1, 2008, Amsterdam, NL). | | | | |

Aufgrund der Messergebnisse wird bestätigt, dass es sich bei dem erfindungsgemäßen Polylactidkomposit um ein Material, das eine Polylactidmatrix (mechanisch stabiles Implantat oder Platzhalter, Knochenersatzmaterial), CaCO₃ zur Abpufferung der bei der Hydrolyse entstehenden Milchsäure und BMP-2 zur Osteoinduktion enthält, handelt, das hervorragend zur Steigerung des Knochenwachstums eingesetzt werden kann.

Die erfindungsgemäße Matrix mit Knochenwachstumsfaktor kann dabei in folgenden Formen vorteilhaft verwendet werden:
- Stifte, Nägel, Schrauben
- Platten, Membranen
- Beschichtung auf Metallimplantaten, die auch mittels lokaler thermischer Behandlung am Applikationsort im Patienten in ihrer Wirksamkeit gesteigert werden können. In allen Fällen mit oder ohne lokale thermische Behandlung am Applikationsort übersteht beispielhaft BMP-2 Temperaturen ohne Denaturierung bis 100 °C.

Die so hergestellten Formkörper können somit mit Hilfe eines Sonic-Welding-Verfahrens bei Implantation in das Gewebe mit diesem verschmolzen werden.

## Patentansprüche

1. Verfahren zur Herstellung eines Produktes mit Polymermatrix, das die folgenden Schritte umfasst:
a. Mischen eines pulverförmigen biologisch abbaubaren Polymers, ausgewählt aus-Polymilchsäure, Polyglykolsäure oder deren Copolymeren, mit einer wässrigen Lösung eines Knochenwachstumsfaktors der BMP-Klasse mit einer Konzentration in der wässrigen Lösung, die 0,5 mg bis 10 mg Knochenwachstumsfaktor pro Gramm an biologisch abbaubarem Polymer im Produkt entspricht, und mit einem pH-Wert von 4 bis 5, oder mit einem pH-Wert von 9,5 bis 10,5 zu einer Schlämme in einer Menge, dass die wässrige Lösung nahezu vollständig von dem pulverförmigen Polymer aufgesaugt wird;
b. Trocknen der in Schritt a) erhaltenen Schlämme;
c. Einbringen des Produktes aus Schritt b) in eine Formgebungsvorrichtung; und
d. Formen des in die Formgebungsvorrichtung eingebrachten Produktes in eine Applikationsform zur Förderung des zellwachstums.

2. Verfahren nach Anspruch 1, bei dem man in schritt a. zusätzlich ein Cytostatikum, Antibiotikum oder Mischungen davon verwendet.

3. Verfahren nach Anspruch 1 oder 2, bei dem man als Knochenwachstumsfaktor BMP-2 verwendet.

4. Verfahren nach einem der vorhergehenden Ansprüche, bei dem man ein pulverförmiges biologisch abbaubares Polymer mit einer Teilchengröße bis 500 µm verwendet.

5. Verfahren nach einem der vorhergehenden Ansprüche, bei dem das pulverförmige biologisch abbaubare Polymer zusätzlich ein pH-Stabilisierungsmittel enthält.

6. Verfahren nach einem der vorhergehenden Ansprüche, bei dem auf ein Gramm des Polymers 1 bis 3,5, vorzugsweise 1,5 bis 2,5 Milliliter der wässrigen Lösung eingesetzt werden.

7. Verfahren nach einem der vorhergehenden Ansprüche, bei dem die in Schritt a) erhaltene Schlämme lyophilisiert wird.

8. Verfahren nach einem der vorhergehenden Ansprüche, bei dem die getrocknete Schlämme in einen Extruder als Formgebungsvorrichtung eingebracht und mittels des Extruders bei einer Temperatur oberhalb der Glasübergangstemperatur des biologisch abbaubaren Polymers und unterhalb der Denaturierungstemperatur des Knochenwachstumsfaktors zu einem Formkörper extrudiert wird.

9. Verfahren nach einem der Ansprüche 1 bis 8, bei dem die getrocknete Schlämme in einen Autoklaven als Formgebungsvorrichtung eingebracht wird, in dem Autoklaven oberhalb der Glasübergangstemperatur des biologisch abbaubaren Polymers und unterhalb der Zersetzungstemperatur des Knochenwachstumsfaktors einer Druckbegasung mit überkritischem Kohlendioxid unterworfen wird, anschließend der Autoklav druckentlastet wird und das erhaltene schaumförmige Produkt aus dem Autoklaven entnommen wird.

10. Formkörper, erhältlich nach dem Verfahren nach Anspruch 8.

11. Schaumförmiges Produkt, erhältlich nach dem Verwahren nach Anspruch 9.

12. Verwendung des Produktes nach Anspruch 10 oder 11 zur Herstellung
a. eines chirurgischen Befestigungsmittels wie Faden, Stift, Nagel, Schraube oder Niete,
b. einer Platte oder Membran, oder
c. zur Beschichtung von Implantaten.

## Claims

1. A method of producing a product having a polymer matrix, which includes the following steps:
a. mixing a biodegradable polymer in powder form, selected from polylactic acid, polyglycolic acid or copolymers thereof, with an aqueous solution of a bone growth factor of the BMP class in a concentration in the aqueous solution, which corresponds to 0.5 mg to 10 mg of bone growth factor per gram of biodegradable polymer in the product, and with a ph-value of 4 to 5 or a ph-value of 9.5 to 10.5 , to form a slurry in such an amount that the aqueous solution is almost completely absorbed by the polymer in powder form;
b. drying the slurry obtained in step a);
c. introducing the product from step b) into a shaping apparatus; and
d. shaping the product introduced into the shaping apparatus into an application form for promoting cell growth.

2. A method according to claim 1 wherein a cytostatic agent, antibiotic or mixtures thereof are used additionally in step a..

3. A method according to claim 1 or 2, wherein BMP-2 is used as bone growth factor.

4. A method according to one of the preceding claims wherein a biodegradable polymer in powder form of a particle size of up to 500 µm is used.

5. A method according to one of the preceding claims wherein the biodegradable polymer in powder form additionally contains a pH-stabilisation agent.

6. A method according to one of the preceding claims wherein 1 to 3.5, preferably 1.5 to 2.5 millilitres of the aqueous solution is used for one gram of the polymer.

7. A method according to one of the preceding claims wherein the slurry produced in step a) is lyophilised.

8. A method according to one of the preceding claims wherein the dried slurry is introduced into an extruder as the shaping apparatus and extruded by means of the extruder at a temperature above the glass transition temperature of the biodegradable polymer and below the denaturing temperature of the bone growth factor, to afford a shaped body.

9. A method according to one of claims 1 to 8 wherein the dried slurry is introduced into an autoclave as the shaping apparatus, subjected in the autoclave to pressure gassing with supercritical carbon dioxide above the glass transition temperature of the biodegradable polymer and below the decomposition temperature of the bone growth factor, then the autoclave is pressure-relieved and the resulting product in foam form is removed from the autoclave.

10. A shaped body, obtainable by the method according to claim 9.

11. A product in foam form, obtainable by the method according to claim 9.

12. Use of the product according to claim 10 or claim 11 for the production of
a. a surgical fixing means such as thread, pin, nail, screw or rivets,
b. a plate or membrane, or
c. for coating implants.

## Revendications

1. Procédé de fabrication d'un produit comportant une matrice polymère, qui comprend les étapes suivantes :
a. mélange d'un polymère biodégradable pulvérulent, sélectionné parmi de l'acide polylactique, de l'acide polyglycolique, ou leurs copolymères, avec une solution aqueuse d'un facteur de croissance osseux de la classe BMP selon une concentration dans la solution aqueuse qui correspond à 0,5 mg à 10 mg de facteur de croissance osseux par gramme de polymère biodégradable dans le produit, présentant une valeur de pH comprise entre 4 et 5 ou une valeur de pH comprise entre 9,5 et 10,5, pour donner une boue dans une quantité telle que la solution aqueuse est presque entièrement absorbée par le polymère pulvérulent ;
b. séchage de la boue obtenue à l'étape a);
c. Introduction du produit de l'étape b) dans un dispositif de modelage ou de moulage ; et
d. mise en forme du produit introduit dans le dispositif de modelage ou de moulage en une forme d'application visant à favoriser la croissance cellulaire.

2. Procédé selon la revendication 1, dans lequel on utilise en outre lors de l'étape a. un cytostatique, un antibiotique ou des mélanges de ceux-ci.

3. Procédé selon la revendication 1 ou 2, dans lequel on utilise BMP-2 en tant que facteur de croissance osseux.

4. Procédé selon l'une des revendications précédentes, dans lequel on utilise un polymère biodégradable pulvérulent présentant une taille de particules allant jusqu'à 500 µm.

5. Procédé selon l'une des revendications précédentes, dans lequel le polymère biodégradable pulvérulent contient en outre un agent de stabilisation du pH.

6. Procédé selon l'une des revendications précédentes, dans lequel, pour un gramme du polymère, on utilise 1 à 3,5, de préférence 1,5 à 2,5, millilitres de la solution aqueuse.

7. Procédé selon l'une des revendications précédentes, dans lequel la boue obtenue à l'étape a) est lyophilisée.

8. Procédé selon l'une des revendications précédentes, dans lequel la boue séchée est introduite dans une extrudeuse en tant que dispositif de modelage ou de moulage, et est extrudée en un corps moulé au moyen de l'extrudeuse, à une température supérieure à la température de transition vitreuse du polymère biodégradable et inférieure à la température de dénaturation du facteur de croissance osseux.

9. Procédé selon l'une des revendications 1 à 8, dans lequel la boue séchée est introduite dans un autoclave en tant que dispositif de modelage ou de moulage, est soumise dans l'autoclave à une injection de gaz sous pression avec du dioxyde de carbone surcritique, à une température supérieure à la température de transition vitreuse du polymère biodégradable et inférieure à la température de destruction du facteur de croissance osseux, puis l'autoclave est déchargé de la pression et le produit moussant obtenu est retiré de l'autoclave.

10. Corps moulé pouvant être obtenu selon le procédé selon la revendication 8.

11. Produit moussant pouvant être obtenu selon le procédé selon la revendication 9.

12. Utilisation du produit selon la revendication 10 ou 11 pour fabriquer
a. un moyen de fixation chirurgical, comme des fils, des chevilles, des clous, des vis ou des rivets,
b. une plaque ou une membrane, ou
c. pour revêtir des implants.
